# EUROPEAN PATENT APPLICATION

(11) **EP 3 878 963 A1**
(43) Date of publication of application: **15.09.2021**
(21) Application number: 19881007.9
(22) Date of filing: 04.11.2019
(51) Int. Cl.: C12N 15/115, A23L 29/00, A23L 33/10, A61K 8/60, A61K 31/7088, A61P 17/00, A61Q 19/00

(54) **METHOD FOR PREVENTING OXIDATION OF POLYPHENOL BY MEANS OF APTAMER, MATERIAL THEREOF, AND USE THEREOF**

(30) Priority: 06.11.2018 KR 20180135214
(71) Applicant: Nexmos Co., Ltd., Gyeonggi-do 16827 (KR)
(72) Inventor: SON, In Sik, Seongnam-Si Gyeonggi-do 13614 (KR)
(74) Representative: FRKelly
(86) International application number: PCT/KR2019/014801
(87) International publication number: WO 2020/096296

(57) **Abstract**

The present invention relates to a method for preventing oxidation of polyphenols, materials thereof, and uses thereof, and more particularly, it relates to a method of preventing oxidation of catechins by treating catechins with aptamers, aptamers having such activity with preventing oxidation of catechins, and it relates to its application to various fields such as cosmetics, foods, and pharmaceuticals using the aptamer. The aptamer of the present invention has an antioxidant effect of catechins, so the aptamer of the present invention can be applied in various fields such as cosmetics, foods, and pharmaceuticals that require inhibition of oxidation of catechins.

<110>	Nexmos Co., Ltd.

<120>	A METHOD FOR INHIBITING OXIDATION OF POLYPHENOL USING APTAMER, A
	MATERIAL THEREFOR, AND USE THEREOF

<130>	OP19-0031HSPCT

<150>	KR 10-2018-0135214
<151>	2018-11-06

<160>	9

<170>	KopatentIn 2.0

<210>	1
<211>	27
<212>	DNA
<213>	Artificial Sequence

<220>
<223>	aptamer


<400>	1
cgagtgaacg acgaggcgcg tcacact	27


<210>	2
<211>	33
<212>	DNA
<213>	Artificial Sequence

<220>
<223>	aptamer


<400>	2
gcacggcaca acgggcgcgc ctccatgctg ttc	33


<210>	3
<211>	29
<212>	DNA
<213>	Artificial Sequence

<220>
<223>	aptamer


<400>	3
gaccaacgga agcgcggcac cacaacggt	29


<210>	4
<211>	30
<212>	DNA
<213>	Artificial Sequence

<220>
<223>	aptamer


<400>	4

cgacgacagg aggtgcggcc ccggcagacc                                        30

<210>    5
<211>    29
<212>    DNA
<213>    Artificial Sequence

<220>
<223>    aptamer

<400>    5
acgcatgccg ggcgcgctcc ctgtcgtcc                                         29

<210>    6
<211>    30
<212>    DNA
<213>    Artificial Sequence

<220>
<223>    aptamer

<400>    6
cgagtcagtg cgaggcgctc ccctgtcggt                                        30

<210>    7
<211>    30
<212>    DNA
<213>    Artificial Sequence

<220>
<223>    aptamer

<400>    7
ggtctgccgg ggccgcacct cctgtcgtcg                                        30

<210>    8
<211>    30
<212>    DNA
<213>    Artificial Sequence

<220>
<223>    aptamer

<400>    8
cgaacagcat ggaggcgcgc ccgttgtgcc                                        30

<210>    9
<211>    30
<212>    DNA
<213>    Artificial Sequence

<220>
<223>    aptamer

<400> 9
ggcacgcagt gtgacgcgcc tcgtcgttca 30

## Description

### [Technical Field]

The present invention relates to a method for preventing oxidation of polyphenol using an aptamer, a material thereof, and a use thereof.

### [Background Art]

Polyphenols are secondary metabolites of plants, including flavonoids and tannins, and are widely distributed in nature, and are contained in many fruits and teas. Currently, polyphenols is known as anti-caries, angiotensin-converting enzyme inhibitory activity related to hypertension, mutagenicity inhibitory activity involved in the initial process of carcinogenesis, inhibition of tartar-forming enzyme, and inhibition of the production of organic acids by bacteria in the oral cavity, as well as antioxidant activity. Among the polyphenols, a number of components exhibiting functions such as bio-protection and bioregulation, which are attracting attention as the tertiary function of food, have also been found.

Among polyphenols, catechin is a compound present in most fruits. It acts as a free radical scavenger in the process of fatty acidization. It is known to act as a cause of enzymatic browning by polyphenol oxidase. Flavonoids, catechins, and phenolic compounds act as secondary metabolites, and condensed tannins are formed by collecting polymerized catechins. Condensed tannins exhibit various physiological activities depending on the degree of polymerization of catechins, and the physiological and pharmacological actions studied so far are reported to be effective in anti-allergic, antioxidant, anti-tumor, anti-cancer, tooth decay prevention, and relieving arteriosclerosis. In addition, catechins contained in green tea have anti-mutagenicity, which is thought to inactivate carcinogenic substances by removing free radicals by combining the -OH group of the polyphenol component with an unstable group having carcinogenicity. Regarding the antibacterial activity, it has been reported that the polyphenol component of tea exhibits antibacterial activity against Clostridium botulinum and food poisoning bacteria, and the catechin component contained in tea exhibits antibacterial activity against Streptococcus mutans, which is the causative agent of tooth decay.

Catechin is derived from the tea family (Camellia sinensis), and is included in white tea, green tea, black tea, and oolong tea. The major catechins are epigallocatechin gallate (EGCG), epigallocatechin (EGC), epicatechin gallate (ECG), and epicatechin (EC).

Catechin has various effects such as carcinogenesis, arteriosclerosis, suppressing blood pressure increase, blood clot prevention, antiviral, anti-obesity, anti-diabetes, antibacterial, detoxification, anti-inflammatory, tooth decay prevention, prevention of polydipsia, and normalization of the intestinal flora. It has been reported that the antioxidant activity of catechins and the free radical removal function, which is a fundamental element of aging, has a stronger free radical removal effect than vitamin C and vitamin E, and has a cardiovascular protection effect such as low density lipoprotein oxidation due to its strong antioxidant activity. Catechins have been shown to stop or slow the production and initiation of undesirable cell populations, and to block those that empirically cause DNA damage.

Antioxidants such as polyphenols and catechins are used to enhance the aforementioned therapeutic or cosmetic performance. However, in order for antioxidants to be effective, they must be maintained in their unoxidized form. As a result, there is a need to maintain antioxidant stability in formulations suitable for administration.

### [Prior patent literature]

Korean Patent Publication No. 10-2015-0143698

### [Disclosure]

### [Technical Problem]

The present invention solves the above problems and is due to the necessity of the above, an object of the present invention is to provide a method for preventing oxidation of polyphenols including catechins.

Another object of the present invention is to provide a material that acts to prevent oxidation of polyphenols including catechins.

Another object of the present invention is to provide a use as a cosmetic product using a substance that acts as an antioxidant for polyphenols including catechins.

Another object of the present invention is to provide a material that maintains a reduced state of polyphenols including catechins to maintain its antioxidant function for a long time.

Another object of the present invention is to provide foods and beverages and foods through a method of maintaining a reduced state of polyphenols including catechins in a liquid such as water for a long period of time.

Another object of the present invention is to provide a use as a drug by using a substance that prevents oxidation of polyphenols including catechins.

### [Technical Solution]

In order to achieve the above object, the present invention provides a method of reducing the oxidation rate of catechin by treating an aptamer on a polyphenol, for example, catechin, thereby maintaining its reducing activity.

In one embodiment of the present invention, the catechin is preferably a material selected from the group consisting of catechin (C), epicatechin (EC), gallocatechin (GC), epigallocatechin (EGC), catechin gallate (CG), epicatechin gallate (ECG), gallocatechin gallate (GCG), and epigallocatechin gallate (EGCG), but is not limited thereto.

In one embodiment of the present invention, the aptamer is preferably composed of one nucleotide sequence selected from the group consisting of the nucleotide sequences of SEQ ID NOs: 1 to 9, but in addition to the aptamer, it is proven through the examples of the present invention. Likewise, all aptamers having different sequences and achieving the desired effect of the present invention are included in the protection scope of the present invention.

In the present invention, aptamin K refers to a complex of epigallocatechin gallate (EGCG) and aptamer.

In addition, the present invention provides an aptamer that prevents oxidation of catechins.

In addition, the present invention provides a cosmetic composition comprising the aptamer of the present invention as an active ingredient.

In addition, the present invention provides a food composition comprising the aptamer of the present invention as an active ingredient.

In one embodiment of the present invention, the food is preferably a food selected from the group consisting of beverages, confectionery, candy, dairy products, gum, paste, bread, and ice cream, but is not limited thereto.

In addition, the present invention provides a pharmaceutical composition comprising the aptamer of the present invention as an active ingredient.

In the present invention, a "polyphenol or polyphenol-based compound" is known to those skilled in the art as a molecule having more than one phenol group. Polyphenols include epigallocatechin, gallate, epi- or gallochatechin, baicalin, taxifolin, quercetin, and hesperetin. hesperetin) or a flavonoid polyphenol such as genistein. Alternatively, the polyphenol can be a non-flavonoid polyphenol. For example, resveratrol is a polyphenol, not a flavonoid.

The phrases "polyphenolic antioxidant" and "polyphenolic compound" are often used interchangeably because most polyphenolic compounds have antioxidant properties.

As used herein, substances referred to as "antioxidants", when present in a mixture containing oxidizable substrate biological molecules, significantly retard or prevent oxidation of the substrate biological molecules. Antioxidants remove biologically important reactive free radicals or other reactive oxygen species (O2-, H2O2, OH, HOCl, ferryl, peroxyl, peroxynitrile and alkoxyl), or It acts by preventing its formation or by catalytically converting free radicals or other reactive oxygen species into less reactive species.

### 1. Aptamer-based cosmetic application example

The present invention provides an aptamer that reduces the rate of oxidation of polyphenols (for example, catechins).

The present invention provides a cosmetic composition comprising an aptamer and an additional component to reduce the oxidation rate of polyphenol (for example, catechin).

The specific ingredient of the present disclosure may be any raw material used in cosmetics, regardless of the kind of extracts or active ingredients. Examples thereof may include various extracts such as, for whitening, a green tea extract, a licorice extract, a mulberry extract, a mulberry root extract, a golden extract, a pueraria extract, a red ginseng extract, for preventing aging, an apricot extract, an oil extract, an orange extract, a lemon extract, a bamboo extract, a guava extract, a rosemary extract, a cornus officinalis extract, a lingshi mushroom extract, a ginkgo extract, a gleditschia australis thorn extract, a paeonia lactiflora root extract, for moisturizing, a quince extract, a white lotus flower extract, a paprika extract, an aloe extract, a cylindrica extract, a seaweed extract, for anti-oxidation, a carrot extract, a soybean extract, a grapefruit seed extract, a grape seed extract, a portulaca oleracea extract, for improving wrinkles, caviar, pomegranate, a ginseng extract, for skin reproduction, a peach extract, a cnidium officinale MAKINO extract, for treating atopy, a centella asiatica extract, a chamomile extract, an adriatic root extract, a sophora flavescens extract, an angelica extract, for treating acne, a peppermint extract, a saururus chinensis extract, a heartleaf houttuynia extract, a peony extract, for anti-inflammatory or anti-bacteria, pyrolignous liquor, a dandelion extract, a calendula extract, a phellodendron amurense extract, a trifoliate orange extract, a golden extract, a fennel extract, a compuri extract, for shrinking pores, a castanea crenata shell extract, a green tea extract, for moisture, glycerin, panthenol, hyaluronic acid, ceramide, beta-glucan, for whitening, albutin, vitamin C, whitense, retinol, astaxanthin, resveratinol, polyphenol, for elasticity, elastin, collagen, coenzyme Q10, effectin, EGF, for anti-infective anti-bacterial agent, propolis, allantoin, phytostan, infra acid, antioxidant vitamin E (natural tocopherol) ROE (a rosemary oil extract), a grapefruit seed extract.

Suitable cosmetic formulations of the present invention may be provided in the form of, for example, solutions, gels, semi-solid or solid creams, skins, lotions, powders, ointments, sprays or conceal sticks. In addition, it may be prepared in the form of a foam or in the form of an aerosol composition further containing a compressed propellant.

In addition, the cosmetic composition of the present invention may further include fatty substances, solubilizers, thickeners and gelling agents, softeners, antioxidants, suspending agents, stabilizers, foaming agents, fragrances, surfactants, water ionic or nonionic emulsifiers, fillers, sequestering and chelating agents, preservatives, vitamins, blockers, wetting agents, essential oils, dyes, pigments, hydrophilic or lipophilic actives, lipid vesicles or adjuvants commonly used in the field of cosmetics or dermatology. In addition, the above components may be introduced in an amount generally used in the field of dermatology.

The cosmetic composition of the present invention can be prepared in any formulation conventionally prepared in the art, and specific formulations thereof may include formulations such as skin lotion, skin softener, skin toner, astringent, lotion, milk lotion, moisturizer lotion, nutrition lotion, massage. cream, nutritional cream, moisturizer cream, hand cream, essence, nutritional essence, pack, soap, shampoo, cleansing foam, cleansing lotion, cleansing cream, body lotion, body cleanser, and bath aid, etc.

### 2. Food and beverage and food composition using aptamer and/or catechin complex

The present invention provides a food composition or a beverage composition comprising the aptamer of the present invention alone or a complex of an aptamer and a polyphenol (for example, catechin) as an active ingredient.

In one embodiment of the present invention, the composition preferably further comprises one or more of collagen, elastin, hyaluronic acid, and peptide, but is not limited thereto.

In one embodiment of the present invention, the food composition is preferably confectionery, candy, dairy, gum, paste, bread, or ice cream, but is not limited thereto.

### 3. Pharmaceutical composition using aptamer and catechin complex

In case that the composition of the present invention is a pharmaceutical composition, it can be used in an indication showing a pharmacological effect related to the antioxidant of polyphenols such as catechins mentioned in the background art. For administration of the composition of the present invention, a pharmaceutically acceptable carrier, excipient, or diluent may be included in addition to the above-described active ingredients. Examples of the carrier, excipient and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl-hydroxybenzoate, propyl-hydroxybenzoate, talc, magnesium stearate, and mineral oils.

The pharmaceutical compositions of the present invention can be formulated and used in the form of oral dosage forms such as powders, granules, tablets, capsules, suspensions, emulsions, syrups; external preparations such as aerosols, etc., suppositories, or sterile injectable solutions according to a conventional method. Specifically, when formulated, it may be prepared using a diluent or excipient such as a filler, a weight agent, a binder, a wetting agent, a disintegrant, a surfactant, etc. that are commonly used. Solid preparations for oral administration include, but are not limited to, tablets, pills, powders, granules, capsules, and the like. Such a solid preparation may be prepared by mixing at least one excipient, for example, starch, calcium carbonate, sucrose, lactose, gelatin, etc. in addition to the active ingredient. In addition, in addition to simple excipients, lubricants such as magnesium stearate and talc may also be used. It can be prepared by adding various excipients, such as wetting agents, sweetening agents, fragrances, preservatives, and the like, in addition to oral liquids and liquid paraffins. Formulations for parenteral administration include sterilized aqueous solutions, non-aqueous solvents, suspensions, emulsions, lyophilized formulations. As the non-aqueous solvent and suspending agent, propylene glycol, polyethylene glycol, vegetable oils such as olive oil, injectable esters such as ethyl oleate, and the like may be used. As a base for suppositories, witepsol, macrosol, Tween 61, cacao butter, laurin, glycerol-gelatin, and the like may be used.

A suitable dosage of the pharmaceutical composition of the present invention varies depending on the condition and weight of the patient, the severity of the disease, the form of the drug, and the time, but may be appropriately selected by a person skilled in the art, and the daily dosage of the composition is preferably 0.001 mg/kg body weight to 500 mg/kg body weight, and it can be administered once or several times a day as needed.

### [Advantageous Effects]

As can be seen through the present invention, the aptamer of the present invention has an effect of inhibiting oxidation of catechin, and using the aptamer selectively binding to catechin of the present invention alone or a complex of aptamer and catechin, it can be used in functional cosmetics of various formulations by maintaining reduced state of catechin and maintaining its antioxidant function for a long time. By using an aptamer that selectively binds to catechins, it is possible to expect sustained and maximized antioxidant effects even with small amounts of catechins. In addition, as can be seen from the present invention, the present invention maintains a reduced state of physiologically active ingredients such as catechins by using an aptamer that selectively binds to catechins to maintain its antioxidant function for a long period of time, so that it can be used for various health beverages, antioxidant beverages and antioxidant food, etc.

In addition, preventing the oxidation of catechins using an aptamer is a safe and innovative approach to a new concept compared to the existing method, and it can be manufactured to exhibit effects by applying it to various industries. In particular, it will be a catalyst that transforms the existing chemical-based cosmetics and food market into a DNA (Bio)-based market. In the future, it is expected to provide an explosive increase and innovative solutions in the DNA market.

### [Description of Drawings]

Figure. 1 is a graph showing the effect of delaying EGCG oxidation in aptamer K1 (SEQ ID NO: 1) to K9 (SEQ ID NO: 9) of the present invention. Nine types of aptamer K from aptamer K1 to aptamer K9 that bind to EGCG were prepared in the same manner as described above. After 20 µM aptamer K1 to K9 and 500 µM EGCG were reacted for 30 minutes, respectively, WST-1 assay was performed. The degree of delaying EGCG oxidation of each aptamer K was compared.

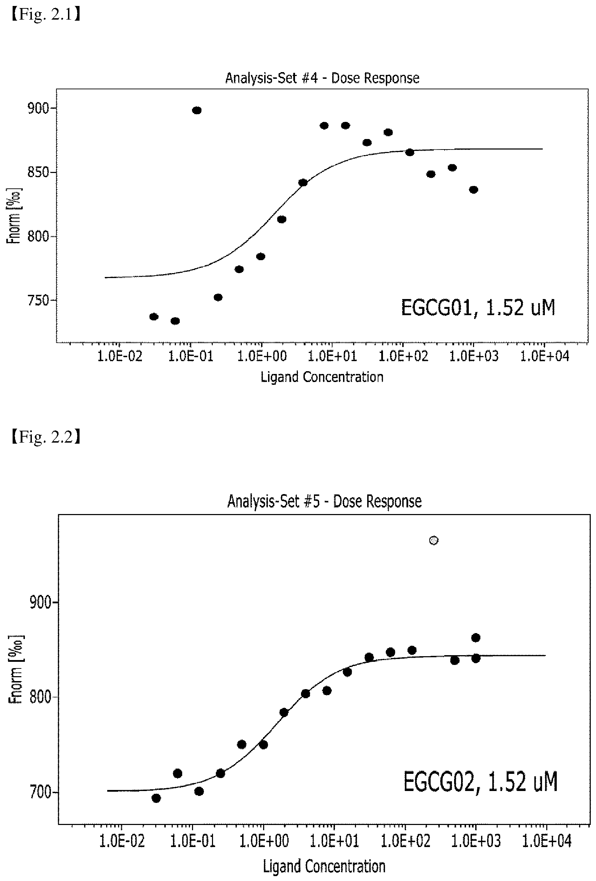

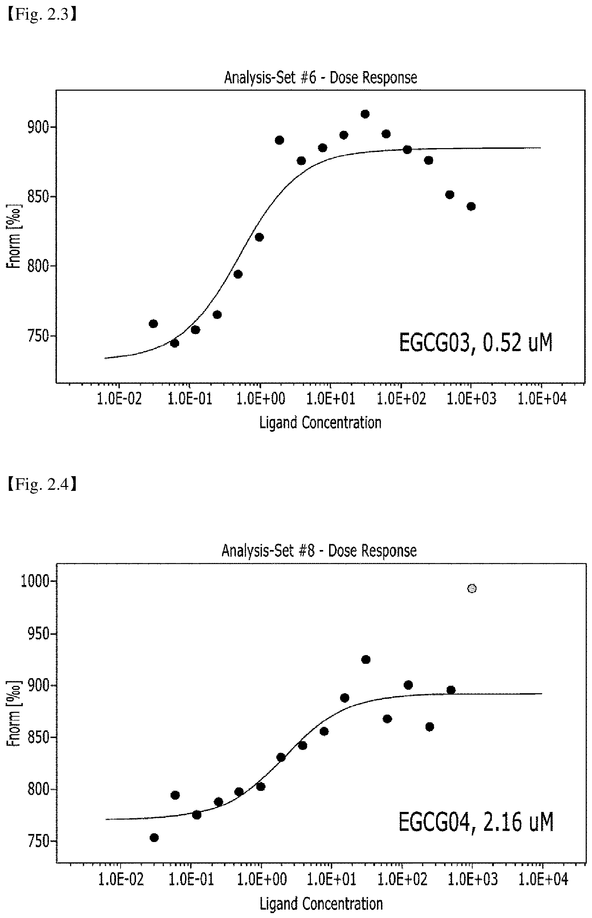

Figure 2 is a graph of the dissociation constant (K_{D}) for EGCG of the aptamer of the present invention.
Figures 3 and 4 are graphs showing the effect of the aptamers K4 and K5 of the present invention on EGCG oxidation delay, as can be seen from the figure, in the case of EGCG, it is rapidly oxidized and reduced WST-1, but In the case of EGCG + aptamer K4 or K5, it was confirmed that the oxidation of EGCG was delayed depending on the concentration and the reduction of WST-1 was delayed.
Figure 5 is a graph comparing the effects of aptamers K4 and K5 of the present invention on EGCG oxidation delay.

### [Mode for Invention]

Hereinafter, the present invention will be described in more detail by the following examples. However, the following examples are described with the intention of illustrating the present invention, and the scope of the present invention is not to be construed as being limited by the following examples.

### Example 1: DNA aptamer selection and sequence analysis

### EGCG SELEX:

Eight rounds of SELEX against EGCG were performed using a DNA library consisting of -10¹⁵ unique oligonucleotides.

**[Table 1]**

| Name | Sequence (5'→3') | No. of mer |
|---|---|---|
| SEQ ID NO: 1 | CGAGTGAACGACGAGGCGCGTCACACT | 27 |
| SEQ ID NO: 2 | GCACGGCACAACGGGCGCGCCTCCATGCTG TTC | 33 |
| SEQ ID NO: 3 | GACCAACGGAAGCGCGGCACCACAACGGT | 29 |
| SEQ ID NO: 4 | CGACGACAGGAGGTGCGGCCCCGGCAGAC C | 30 |
| SEQ ID NO: 5 | ACGCATGCCGGGCGCGCTCCCTGTCGTCC | 29 |
| SEQ ID NO: 6 | CGAGTCAGTGCGAGGCGCTCCCCTGTCGGT | 30 |
| SEQ ID NO: 7 | GGTCTGCCGGGGCCGCACCTCCTGTCGTCG | 30 |
| SEQ ID NO: 8 | CGAACAGCATGGAGGCGCGCCCGTTGTGCC | 30 |
| SEQ ID NO: 9 | GGCACGCAGTGTGACGCGCCTCGTCGTTCA | 30 |

Table 1 is a table showing the aptamer sequence specifically binding with EGCG.

### Example 2: Effect of the aptamer of the present invention on EGCG oxidation delay

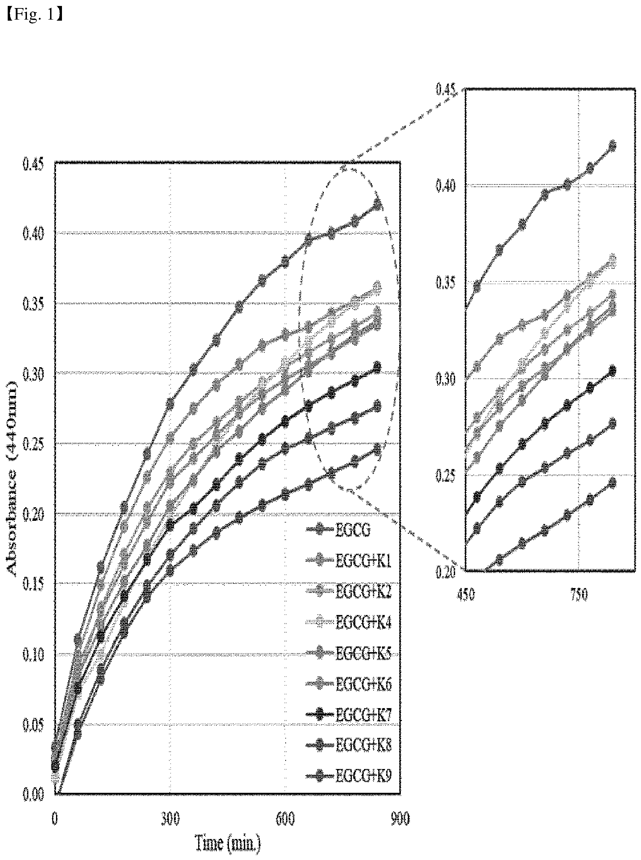

The effect of the aptamers K1 to K9 of the present invention on EGCG oxidation delay was performed in the same manner as in Example 2, and the results are shown in FIG. 1.

As can be seen from FIG. 1, when K1 to K9 aptamer was treated, a significant effect of delaying EGCG oxidation of about 30-40% was observed.

The aptamer (aptamers of SEQ ID NOs: 1 to 9) of the present invention reacting specifically with the EGCG obtained through the SELEX was dissolved in a folding buffer (1mM MgCl2 in PBS) and then dissolved at 95°C. After boiling for a minute, the temperature was gradually lowered to room temperature to form a tertiary structure.

After reacting 500 uM EGCG and one of the prepared 20 µM aptamers K1 to K9 and for 30 minutes, WST-1 assay was performed. The degree of EGCG oxidation delay of each aptamer K was compared.

As can be seen from FIG. 1, the group treated with one of the aptamers of K1 to K9 exhibited about 20% to 40% of the EGCG oxidation delay effect compared to the control without treatment.

### Example 3: Effect of Aptamer K4 on EGCG Oxidation Delay

Aptamer K4, which specifically reacts with EGCG obtained through SELEX, was dissolved in folding buffer (1mM MgCl2 in PBS), boiled at 95° C. for 5 minutes, and then gradually lowered to room temperature to form a tertiary structure.

500 mM EGCG and 5, 10, and 20 uM of the prepared aptamer K4 were reacted at room temperature for 30 minutes, respectively, and then WST-1 assay was performed.

Prepared sample and WST-1(3-(4,5-Dimethylthiazol-2-yl)-2,5-diphenyltetrazolium bromide) was reacted to measure the formazan produced and with the plate reader, 440nm absorbance was measured for 16 hours.

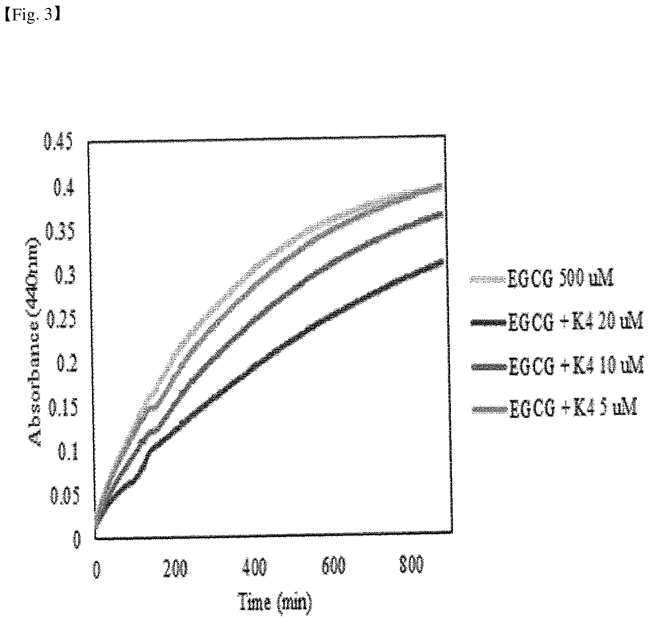

In the case of EGCG, it was rapidly oxidized and reduced WST-1, but in the case of EGCG + aptamer K4, the oxidation of EGCG was delayed (about 33% at 20 uM) depending on the concentration of aptamer K4, indicating that the reduction of WST-1 was delayed (Fig. 3).

### Example 4: Effect of Aptamer K5 on EGCG Oxidation Delay

The aptamer K5 (aptamer of SEQ ID NO: 5) that specifically reacts with EGCG obtained through the SELEX was prepared in the same manner as described above and was tested for the effect of delaying EGCG oxidation.

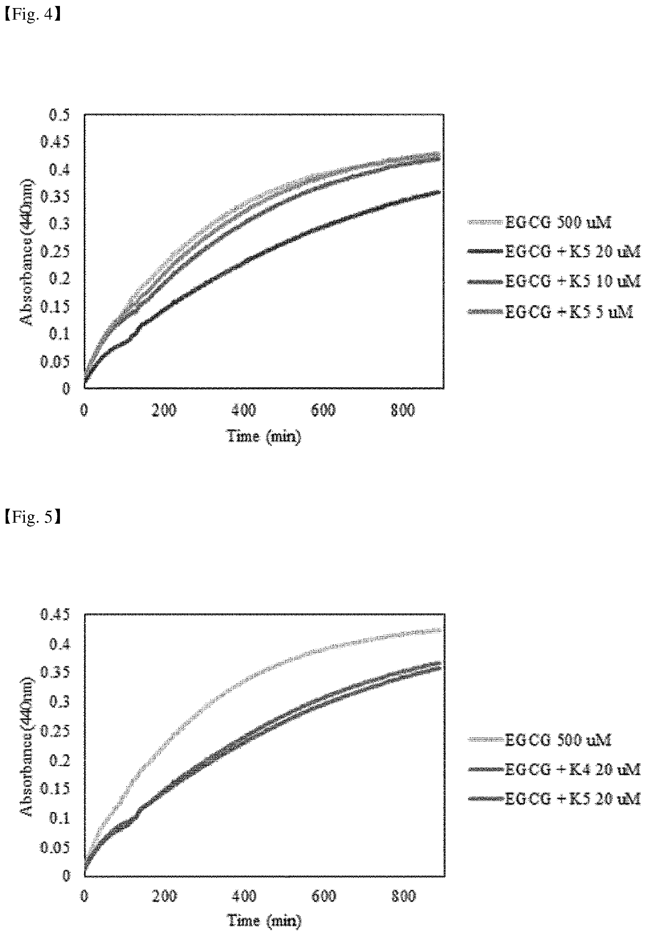

In the case of EGCG, it was rapidly oxidized and reduced WST-1, but in the case of EGCG + aptamer K5, the oxidation of EGCG was delayed (about 33% at 20 uM) depending on the concentration of aptamer K5, indicating that the reduction of WST-1 was delayed (Fig. 4).

### Example 5: Comparison of the effect of aptamers K4 and K5 on EGCG oxidation delay

Aptamers K4 and K5 binding to the EGCG were prepared in the same manner as described above. 20 µM aptamer K4, or aptamer K5, and 500 µM EGCG were reacted for 30 minutes, respectively, followed by WST-1 assay. It was confirmed that the EGCG oxidation delay effect of 20 µM aptamer K4 and aptamer K5 was similar (FIG. 5).

## Claims

1. A method of preventing oxidation of catechins by treating aptamers with catechins.

2. The method of claim 1, wherein the catechin is one of the material selected from the group consisting of catechin (C), epicatechin (EC), gallocatechin (GC), epigallocatechin (EGC), catechin gallate (CG), epicatechin gallate (ECG), gallocatechin gal. Rate (GCG) and epigallocatechin gallate (EGCG).

3. The method of claim 1, wherein the aptamer consists of one nucleotide sequence selected from the group consisting of nucleotide sequences of SEQ ID NOs: 1 to 9.

4. An aptamer that prevents oxidation of catechins.

5. The aptamer of claim 4, wherein the catechin is one of the material selected from the group consisting of catechin (C), epicatechin (EC), gallocatechin (GC), epigallocatechin (EGC), catechin gallate (CG), epicatechin gallate (ECG), gallocatechin gal. Rate (GCG) and epigallocatechin gallate (EGCG).

6. The aptamer of claim 4, wherein the aptamer consists of one nucleotide sequence selected from the group consisting of nucleotide sequences of SEQ ID NOs: 1 to 9.

7. A cosmetic composition comprising the aptamer of claim 4 as an active ingredient.

8. The cosmetic composition according to claim 7, wherein the aptamer comprises one nucleotide sequence selected from the group consisting of nucleotide sequences of SEQ ID NOs: 1 to 9.

9. A food composition comprising the aptamer of claim 4 as an active ingredient.

10. The food composition according to claim 9, wherein the aptamer consists of one nucleotide sequence selected from the group consisting of nucleotide sequences of SEQ ID NOs: 1 to 9.

11. The food composition according to claim 9, wherein the food is a food selected from the group consisting of beverages, confectionery, candy, dairy products, gum, paste, bread, and ice cream.

12. A pharmaceutical composition comprising the aptamer of claim 4 as an active ingredient.

13. The pharmaceutical composition according to claim 12, wherein the aptamer consists of one nucleotide sequence selected from the group consisting of nucleotide sequences of SEQ ID NOs: 1 to 9.
